# EUROPEAN PATENT SPECIFICATION

(11) **EP 4 473 639 B1**
(45) Date of publication and mention of the grant of the patent: **22.10.2025**
(21) Application number: 23702841.0
(22) Date of filing: 06.02.2023
(51) Int. Cl.: H02J 50/00, H02J 50/10, A61N 1/378

(54) **CHARGING DEVICE**
LADEVORRICHTUNG
DISPOSITIF DE CHARGE

(30) Priority: 04.02.2022 EP 22155296
(43) Date of publication of application: 11.12.2024
(73) Proprietor: Indigo Diabetes N.V., 9052 Gent (BE)
(72) Inventor: DELBEKE, Danaë, 9050 Gent (BE)
(74) Representative: Winger
(86) International application number: PCT/EP2023/052884
(87) International publication number: WO 2023/148389

(56) References cited:
- US-B2- 9 270 134

## Description

### Technical field of the invention

The present invention relates to field of medical devices. More specifically, the present invention relates to charging devices and systems for implantable sensing devices, such as for example metabolite sensing devices like glucose sensing devices..

### Background of the invention

Implantable devices are more and more used for medical applications. Implantable devices may facilitate continuous detection of subcutaneous analytes such as blood or bodily fluid analytes, e.g., metabolites. As such, implantable devices may enable more accurate and close monitoring, enabling, for example, a person with diabetes to be warned when hyperglycemia or hypoglycemia is imminent, and possibly act so as to prevent said hyperglycemia or hypoglycemia. Furthermore, in particular when frequent detection of analytes is required, implantable devices may provide a huge comfort to the user, as frequent blood drawing may no longer be required.

As the implantable device is located subcutaneously, an external control device, located supracutaneously, may be communicatively, e.g., wirelessly, coupled with the implantable device for transferring data between the external control device and the implantable device. Thereby, data about concentrations of analytes detected by the implantable device may be transferred from the implantable device to the external control device, which may subsequently be displayed to the user.

In the state of the art, charging of the external control devices and of the implantable devices may result in temporary interruption of data transfer. There is thus still a need in the art for devices and methods that address at least some of the above problems. Document US9270134B2 discloses a charging device capable of charging an implantable sensor.

### Summary of the invention

It is an object of the present invention to provide a good apparatus and methods for charging of external control devices and implantable devices.

The above objective is accomplished by a method and apparatus according to the present invention.

It is an advantage of embodiments of the present invention that it enables simultaneous charging of an implantable device and of an external control device. It is a further advantage of embodiments of the present invention that activity of the implantable device and of the external control device can be maintained during charging. It is still a further advantage of embodiments of the present invention that the simultaneous charging facilitates continuous close proximity between the implantable device and the external control device. It is still another advantage of embodiments of the present invention that continuous transfer of data between the implantable device and the external control device may be facilitated, even during charging of both the implantable device and the external device. For example, analyte concentrations in the blood of a user may be continuously monitored and provided, e.g., displayed, to the user, even during charging.

It is an advantage of at least some embodiments of the present invention that charging can be done in a safe manner.

In a first aspect, the present invention relates to a wearable system comprising a charging device for simultaneously charging an implantable device when implanted and an external control device for reading out and/or controlling the implantable device. The charging device comprises: a first charging element, such as for example a charging coil, configured for charging the implantable device e.g. for generating a charging field for transferring power to the implantable device; a second charging element for charging the external control device, e.g. an electrical output element suitable for transferring electrical power to an electrical input element of the external control device. The charging device also comprises at least one electrical power source, configured for providing power via the first charging element and second charging element. In some embodiments, the first charging element is a charging coil and the implantable device comprises a receiving coil adapted to be inductively coupled to each other, so that inductive charging of the implantable device by the charging device may be performed.
The at least one electrical power source itself is chargeable and the system is configured to allow mutually exclusive the charging of the at least one electrical power source on the one hand, and the charging of the implantable sensor and the external control device through the at least one electrical power source on the other hand. The latter provides for improved safety in use, since the at least one electrical power source is not chargeable, for example not connected to an external voltage supply, when the belt is used for charging the implantable sensor. One of the safety aspects is that the charging connector of the electrical power source is mechanically/physically covered so that there is no access thereto when the external control device is in position.

In advantageous embodiments, the system may be configured such that positioning the external control device with respect to the second charging element prevents from charging the at least one electrical power source. This allows for improved safety. In embodiments, the external control device comprises: the electrical input element, a wireless data receiver, and a first rechargeable electrical power source for providing power to the wireless data receiver. Herein, the charging device and the external control device are configured for, when the electrical input element is coupled to the electrical output element for transferring power from the electrical output element to the electrical input element, charging of the first rechargeable electrical power source by the charging device. In embodiments, the wearable system comprises the external control device.

In embodiments, the second charging element, e.g. the electrical output element, and the electrical input element of the external control device are adapted for electrically, e.g., physically, contacting each other. Charging through electrical contacting is typically efficient. In different embodiments, the electrical input element and the electrical output element each comprise a coil, and are adapted for transferring power through inductive coupling. In these embodiments, the charging of the external control device by the charging device may be based on inductive charging. In some embodiments, the electrical output element comprises the charging coil, e.g., the electrical output element is the charging coil. In these embodiments, the charging coil may be configured for simultaneously charging the implantable device and the external control device. Thereby, an efficient charging device, requiring only a limited amount of material and electrical connections, may be obtained. In different embodiments, the electrical output element is different form the charging coil. For example, in different embodiments charging can be performed directly via electrical contacts, can be performed via wireless interaction, can be performed optically, etc.

In some embodiments, the implantable device comprises the receiving coil, a sensor configured for detecting an analyte and for generating data indicative of the presence of the analyte, a wireless data transmitter configured for wirelessly transmitting the data, and a second rechargeable electrical power source for providing power to the sensor and the wireless data transmitter. In these embodiments, the charging device and the implantable device are configured for, when the charging coil is within a suitable distance for transferring power to the receiving coil, charging of the second rechargeable electrical power source by the charging device. In embodiments, the wearable system comprises the implantable device. In some embodiments, alternative or in addition to a sensor for sensing analytes, another type of device such as for example a sensor for measuring hearth rhythm, temperature or another property of the body could be used. Further alternatives or additions may be neurostimulators, active orthopedics, rechargeable implants, and alike.

The wearable system at least comprises the charging device, and may further comprise at least one of, such as both, the implantable device and the external control device. In embodiments, the wearable system is configured for, when simultaneously charging the implantable device and the external device, orienting the external device so as to enable communicative coupling between the external device and the implantable device, that is, simultaneous with the simultaneous charging. Nevertheless, alternatively, interleaved charging also may be used.

In embodiments, the wearable device further comprises wearing means, e.g., a belt, wearable by a user, clipping means for clipping the device on clothes or other items, pinning means for pinning the device on clothes or other items, wherein the wearing means is suitable for holding the charging device. In embodiments, the wearable system is configured for physically contacting the charging device with the user when wearing the wearing means, e.g., the belt, holding the charging device. It is an advantage of these embodiments that the charging device may be as proximate to an implanted implantable device as possible while still being located supracutaneously, which may result in efficient charging of the implantable device by the charging device. The wearing means is preferably a belt, which may provide ease of putting on and bearing and may provide robust fixing of the charging device to the body of the user. The belt may, for example, comprise OEKO-TEX hypoallergenic fabric, which is stretchable and provides comfort to a user by being a breathing material. However, the wearing means is not limited thereto, and may, for example, instead be a piece of cloth such as a shirt, or an adhesive, e.g., an adhesive tape, for fixing the charging device to the skin. The wearing means may facilitate the user to walk around while charging.

The charging device may be aligned with respect to the implantable device, for charging of the implantable device, by a user based on the user feeling the location of the implantable device. For this, the user may, for example, use his finger tips. This is possible when the implantable device is tactile, which is typically the case when the implantable device is located proximate to the skin. In embodiments, the charging device, possibly in combination with the implantable device, is configured for detecting whether the charging device is at a location with respect to the implantable device suitable for transferring power from the charging coil to the receiving coil. In embodiments, said detecting may comprise detecting whenever power is efficiently transferred from the charging coil to the receiving coil. For example, a feedback signal may be generated by the charging device or by the implantable device whenever a predetermined charge rate is exceeded. When said feedback signal is generated by the implantable device, the feedback signal may be transferred from the implantable device to the charging device. For example, the implantable device may be configured transmitting the feedback signal to the charging device by modulating the charge rate, sensible by the charging device; or via a data transmitter, e.g., the wireless data transmitter, in the implantable device. The feedback signal may be transmitted to a data receiver in the charging device, or in the external device, e.g., the wireless data receiver, which may forward said feedback signal to the charging device. In embodiments, the charging device is configured for generating a noise or a visual signal, or vibrate when alignment between the charging device and the implantable device is suitable for transferring the power.

In embodiments, the charging device further comprises a receiving element configured for reversibly immobilizing the external control device with respect to the charging device. Herein, the reversibility of the immobilizing may comprise that the external control device may be releasable from the immobilization, for example, by pressing a button for releasing the external control device. In embodiments, the external control device comprises a fastening element configured for mating with, e.g., for being received by, the receiving element of the charging device. Immobilizing the external control device with respect to the charging device facilitates simultaneous charging of the implantable device and the external control device. The receiving element may, for example, be an opening in which the external control device may be received. The opening may comprise, in its walls, retractable projecting elements, wherein each projecting element couples to the external control device. For example, each projecting element may be received by a hole in the external control device. The receiving element may comprise a button configured for retracting said projecting elements so as to release the external control device. Alternatively, the receiving element may comprise rails for slidingly receiving the external control device. In embodiments, the immobilizing comprises coupling, such as inductively coupling or electrically, optically e.g., physically, contacting, the electrical input element to the electrical output element for transferring power from the electrical output element to the electrical input element.

In embodiments, the external control device comprises an alarm element, and a control unit configured for determining, from data received by the wireless data receiver, whether to generate an alarm with the alarm element. When, for example, during charging, data received by the external control device from the implantable device indicates that metabolite levels are outside of predetermined levels, e.g., are too high or too low, or that the battery is low, or that the connection is or was lost, or that a specific property that is sensed is outside a range, or that the system requires certain attention by the user, etc. the alarm signal may be generated to notify the user. Thereby, no continuous attention by the user on the external control device is required, further facilitating the user to perform activities during the charging.

In embodiments where the implantable device comprises a sensor, the sensor of the implantable device is configured for detecting at least one analyte, such as a metabolite, e.g. one or more of acetate, amino acids, ammonia, creatinine, ethanol, glucose, homocysteine, ketones, lactate, lipids, phenyl sulfate, proponic acid, urea, pyruvate, temperature, vitamin B11 and/or B12, and water, the present invention not being limited thereto, or for detecting a parameter of a user such as for example temperature or heart rhythm, etc. The sensor may be any type of sensor suitable for detecting the at least one analyte. In embodiments, the sensor is an optical sensor, comprising a light emitter, and a light detector, configured for detecting light emitted by the light emitter after propagation through a material, e.g., tissue or blood, to be analysed.

Any features of any embodiment of the first aspect may be independently as correspondingly described for any embodiment of any of the other aspects of the present invention.

In a second aspect, the present invention relates to a method for simultaneously charging an implantable device and an external control device by a charging device.
The method comprises positioning the wearable system on the body so that the implantable device is positioned relative to a first charging element of the wearable system for allowing charging of the implantable device.
The method also comprises coupling the external control device to a second charging element of the wearable system for allowing charging of the external control device.
As indicated above, the method also comprises simultaneously charging the implantable device and the external control device through the wearable system. The method further comprises allowing only mutually exclusive the charging of the at least one electrical power source on the one hand, and the charging of the implantable sensor and the external control device through the at least one electrical power source on the other hand.
According to embodiments of the present invention, only when the external control device is positioned in the charger, thus covering the input for charging the electrical power source, the charger will charge the external control device and the implantable device. The latter results in an increased safety, since charging of the electrical power source of the charging system is not possible during charging of the external control device and the implantable device.
The method also may comprise charging at least one electrical power source of the wearable system when the implantable sensor and the external control device is not charged.
In some embodiments, the method comprises: coupling, for transferring power, an electrical input element of the external device to an electrical input element of the charging device, positioning a charging coil of the charging device within a suitable distance of a receiving coil of the implantable device for transferring power to the receiving coil, and operating the charging device so as to simultaneously generate a magnetic charging field with the charging coil, and applying power to the electrical output element. In embodiments, the method may be performed on a wearable device according to embodiments of the first aspect of the present invention.

In some embodiments, the method may further comprise: detecting, with the sensor of the implantable device, information regarding at least one analyte, and transmitting data, based on said information, from a wireless data transmitter of the implantable device to a wireless data receiver of the external control device. The information may be an indication of whether the analyte is detected, or a concentration of the analyte, e.g. one or more of acetate, amino acids, ammonia, creatinine, ethanol, glucose, homocysteine, ketones, lactate, lipids, phenyl sulfate, proponic acid, urea, pyruvate, temperature, vitamin B11 and/or B12, and water, the present invention not being limited thereto. Alternative or in addition thereto, the method may comprise sensing a certain parameter such as for example temperature or heart rhythm or alike, performing neurostimulation, performing actuation or support using active orthopedics, etc.

In embodiments, the sensor detects a concentration of the analyte, and the data is indicative of the concentration of the analyte. In embodiments, the implantable device comprises means for processing signals detected by the sensor. For example, the implantable device may compare a detected concentration to a predetermine value: when the detected concentration is higher than the predetermined value, the implantable device may transmit data indicating that the predetermined value has been exceeded.

In embodiments, the implantable device is located subcutaneously. In embodiments, an interior of the implantable device is hermetically sealed from an environment of the implantable device.

Any features of any embodiment of the second aspect may be independently as correspondingly described for any embodiment of any of the other aspects of the present invention.

In a third aspect, the present invention relates to a method for making the wearable system according to embodiments of the first aspect. The method comprises: obtaining the following components: the first charging element, e.g. charging coil, the second charging element, e.g. electric output element, and the at least one electrical power source; and arranging said components so as to obtain the charging device. In embodiments, the method may comprise obtaining any further components as described in embodiments of the first aspect, and may further comprise arranging said further components so as to obtain the charging device, the external control device, and/or the implantable device.

In one aspect, the present invention also relates to a wearable system comprising a charging device for simultaneously charging an implantable device when implanted and an external control device for reading out and/or controlling the implantable device. The charging device comprises: a first charging element, such as for example a charging coil, configured for charging the implantable device e.g. for generating a charging field for transferring power to the implantable device; a second charging element for charging the external control device, e.g. an electrical output element suitable for transferring electrical power to an electrical input element of the external control device. The charging device also comprises at least one electrical power source, configured for providing power via the first charging element and second charging element. The charging device further comprises a receiving element, such as a slit or a clip or any other suitable element, for mechanically coupling the external control device to the charging device for charging. The external control device may be adapted accordingly to have complementary features so as to also assist in the mechanical coupling of the external control device to the charging device. It is an advantage of embodiments of the present invention that good simultaneous charging of the implantable device and the external control device can be performed. It also is an advantage of embodiments of the present invention that a single charging device can be used for both charging of the implantable device and the external control device, reducing the number of components that are required to be maintained and stored by the user.

Other features may correspond with standard or optional features as described for the other aspects.

It is to be noted that any features of any embodiment of the third aspect may be independently as correspondingly described for any embodiment of any of the other aspects of the present invention.

Particular and preferred aspects of the invention are set out in the accompanying independent and dependent claims. Features from the dependent claims may be combined with features of the independent claims and with features of other dependent claims as appropriate and not merely as explicitly set out in the claims.

Although there has been constant improvement, change and evolution of devices in this field, the present concepts are believed to represent substantial new and novel improvements, including departures from prior practices, resulting in the provision of more efficient, stable and reliable devices of this nature.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

FIG. 1 is a schematic 3D representation of an external control device in accordance with embodiments of the present invention.
FIG. 2 is an exploded view of the an external control device in accordance with embodiments of the present invention.
FIG. 3 is a schematic 3D representation of a charging device in accordance with embodiments of the present invention.
FIG. 4 is an exploded view of a charging device 3 in accordance with embodiments of the present invention.
FIG. 5 is a schematic representation of a wearable system in according with embodiments of the present invention.
FIG. 6 is a schematic representation of a belt coupled to a charging device in accordance with embodiments of the present invention.
FIG. 7 and FIG. 8 illustrate examples of charging systems according to embodiments of the present invention.

In the different figures, the same reference signs refer to the same or analogous elements.

### Description of illustrative embodiments

The present invention will be described with respect to particular embodiments and with reference to certain drawings, but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. The term "comprising" therefore covers the situation where only the stated features are present and the situation where these features and one or more other features are present. The word "comprising" according to the invention therefore also includes as one embodiment that no further components are present. Thus, the scope of the expression "a device comprising means A and B" should not be interpreted as being limited to devices consisting only of components A and B. It means that with respect to the present invention, the only relevant components of the device are A and B.

Similarly, it is to be noticed that the term "coupled", also used in the claims, should not be interpreted as being restricted to direct connections only. The terms "coupled" and "connected", along with their derivatives, may be used. It should be understood that these terms are not intended as synonyms for each other. Thus, the scope of the expression "a device A coupled to a device B" should not be limited to devices or systems wherein an output of device A is directly connected to an input of device B. It means that there exists a path between an output of A and an input of B which may be a path including other devices or means. "Coupled" may mean that two or more elements are either in direct physical or electrical contact, or that two or more elements are not in direct contact with each other but yet still co-operate or interact with each other.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

The invention will now be described by a detailed description of several embodiments of the invention. It is clear that other embodiments of the invention can be configured according to the knowledge of persons skilled in the art without departing from the technical teaching of the invention, the invention being limited only by the terms of the appended claims.

In a first aspect, the present invention relates to a wearable system comprising a charging device for simultaneously charging an implantable device and an external control device for reading out and/or controlling the implantable device. The charging device comprises at least one electrical power source, a first charging element for charging the implantable device and a second charging element for charging the external control device, the charging device configured for simultaneously charging the implantable device and the external control device. The at least one electrical power source itself is chargeable and the system is further configured to only allow mutually exclusive the charging of the at least one electrical power source on the one hand, and the charging of the implantable sensor and the external control device through the at least one electrical power source on the other hand.

Further features and advantages will be described with reference to particular examples of embodiments of the present invention.

According to some embodiments, the wearable system comprises a charging device for simultaneously charging an implantable device comprising a receiving coil, and an external control device comprising an electrical input element. Alternatively, the external control device may comprise a coil for being charged inductively, an optical input element for being charged optically or any other type of element allowing charging of the external control device. The charging device comprises: a charging coil configured for generating a magnetic charging field for transferring power to the receiving coil; an electrical output element suitable for transferring electrical power to the electrical input element; and at least one electrical power source, configured for providing power to the charging coil and to the electrical output element.

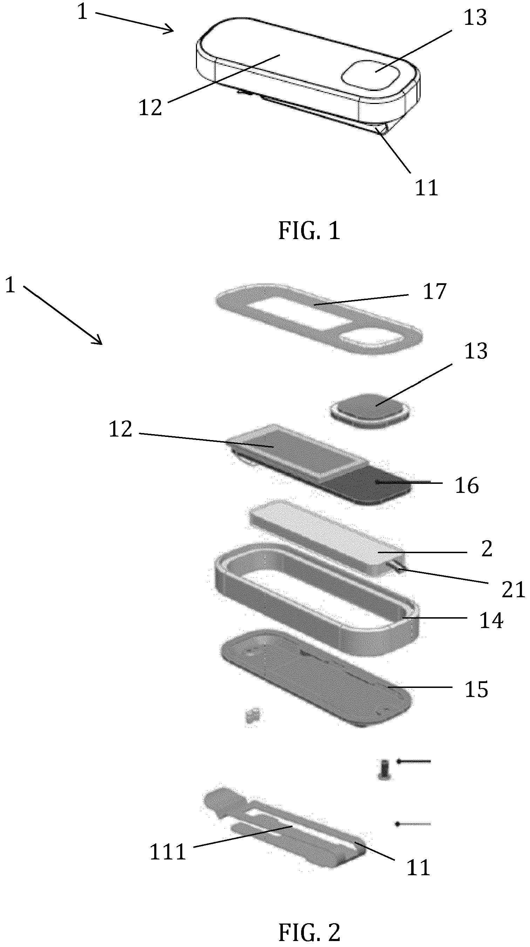

Reference is made to FIG. 1, which is a schematic 3D representation of an external control device 1 in accordance with embodiments of the present invention. The external control device 1 comprises a display 12 for displaying, for example, data about concentrations of analytes, detected by an implantable device (not shown), and transferred from said implantable device to the external control device 1. The external control device 1 may comprise a memory device for saving data transmitted from the implantable device to the external control device. Alternatively, the implantable device may comprise a memory device for saving data measured with a sensor of the implantable device. Based on past data, the display 12 may, for example, indicate, with an arrow, whether metabolite levels are continuous, decreasing, or increasing, over time. Said displaying may be performed also during charging of the external control device 1 and/or the implantable device, for example, alternatingly with a status of said charging. The display 12 may be used for displaying any suitable information such as for example an alarm signal whenever a hyperglycemia or hypoglycemia is imminent or detected in the case of a glucose sensor, a level of a certain analyte, a parameter sensed in the body, an indication of an alarm, an indication of a battery status, an indication of a performed action or activity, etc.

The external control device 1 may, thus, in combination with the implantable device, be used to monitor the concentration of analytes in a body of a user, e.g., of the concentration of glucose present in tissue, interstitual fluid, urine, saliva or blood of the user. It is to be noted that the analyte may be one or more of acetate, amino acids, ammonia, creatinine, ethanol, glucose, homocysteine, ketones, lactate, lipids, phenyl sulfate, proponic acid, urea, pyruvate, temperature, vitamin B11 and/or B12, and water, the present invention not being limited thereto. Said data may be displayed by the display 12 either continuously, or only for some time after pressing a tactile button 13. When the wearable system comprising the external control device 1 is adapted for measuring different types of analytes, said button 13 may be used to toggle through the data for the different types of analytes. Alternatively or in addition, said button 13 may be used to toggle through menus, e.g., to change any settings of the external control device 1 to be in accordance with preferences of the user, or to set up a communicative coupling with the implantable device. In this example, the external control device 1 comprises a clip, e.g., a spring steel clip, for attachment to a user, e.g., to clothes worn by said user. The external control device 1 is typically worn in the proximity of the implantable device, so as to enable communicative coupling between the external control device 1 and the implantable device.

Reference is made to FIG. 2, which is an exploded view of an external control device 1 in accordance with embodiments of the present invention. A printed circuit board 16 is communicatively coupled to a display 12 and a tactile button 13. The printed circuit board 16 is electrically powered by a first rechargeable electrical power source 2. The printed circuit board is connected to the battery via item 21. The external device is connected to the charging device via the two pins shown, for charging of the first rechargeable electrical power source 2 by the charging device. The first rechargeable electrical power source 2 and the printed circuit board 16, and preferably also the display 12, are located in a housing comprising a top casing element 17, a circumfering casing element 14, and a bottom casing element 15. The housing is preferably made of an electrically non-conducting material, i.e., of a material having a high electrical resistance. The top casing element 17 may be made of any material, e.g., gorilla glass, and may comprise an opening for exposing the tactile button 13. The display 12 is optically exposed to the environment through a transparent material in the top casing element 17 or through an opening in the top casing element 17. The circumfering casing element 14 may be made, for example, of 7075-T6 aluminium alloy, SAE 304 stainless steel, or of plastic through plastic injection. The bottom casing element 15 is preferably made of a material transparent to RF radiation, e.g., a plastic, such as medical grade polycarbonate. In this example, the clip 11 comprises a cut out 111, i.e., an opening 111, so as to facilitate a communicative coupling between the wireless data receiver (not shown, but typically present between the bottom casing element 15 and the first rechargeable electrical power source 2) of the external control device 1 and the wireless data transmitter of the implantable device (not shown).

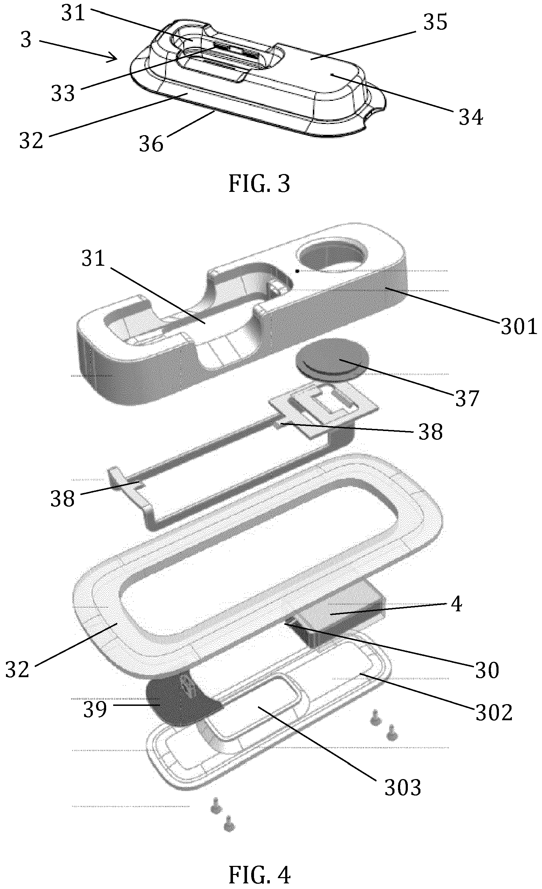

Reference is made to FIG. 3, which is a schematic 3D representation of a charging device 3 in accordance with embodiments of the present invention. In this example, the charging device 3 comprises a receiving element comprising an opening 31 in which an external control device (not shown) may be received. The opening 31 extends from a top surface 35 to a bottom surface 36 of the charging device 3. The bottom surface 36 of the charging device 3 may contact the user, e.g. at the skin or at the clothes. By having the opening 31 extend to the bottom surface 36, the external control device may be located as close as possible to the user, which may result in good inductive coupling between the external control device and an implantable device (not shown).

In this example, the receiving element further comprises rails 33 on walls of the opening 31 for slidingly receiving the external control device. The external control device may, for example, comprise, on the long sides of circumfering casing element 14 in FIG. 1, complementary elements, e.g., complementary rails, alternative projecting elements, or grooves for mechanical coupling with the rails 33. The receiving element may enable the external control device to be immobilized with respect to the charging device 3, e.g., by sliding the complementary elements on the rails 33 of the receiving element. Said immobilization may be reversed, e.g., by sliding the complementary elements from the rails 33 of the receiving element, and removing the external control device from the opening 31 of the charging device 3. The immobilization comprises electrically contacting an electrical input element of the external control device to an electrical output element (obscured in the view of FIG. 3) of the charging device 3, so that the external control device may be charged by the charging device 3. An indicator 34, e.g., a LED, may indicate whether said charging is still being performed, or is complete. Alternatively, a screen of the external control element may indicate the status of charging of the external control element and/or of the implantable device, and the indicator 34 may be for indicating whether the electrical power source of the charging device 3 is low. In this example, the charging device 3 comprises an elastic band 32 for easy fit into a wearing means, e.g., a belt (not shown).

Reference is made to FIG. 4, which is an exploded view of a charging device 3 in accordance with embodiments of the present invention. In this example, the casing of the charging device 3 comprises a top casing element 301 and a bottom casing element 302. The charging device 3 comprises a receiving element comprising an opening 31 for receiving the external control device (not shown), the opening 31 extending through the charging device 3. In this example, the receiving element further comprises retractable projecting elements 38, projecting from walls in the opening 31, wherein each projecting element 38 may couple to the external control device, for example, by being received by a hole in the external control device. Said holes may, for example, be located in the short sides of circumfering casing element 14 in FIG. 1. In this example, the receiving element is adapted for retracting the retractable projecting elements 38 by pressing a button 37, thereby decoupling the receiving element from the external control device, which may then be removed from the opening 31. Although, in this example, the button is located in a top surface of the charging device 3, alternatively, said button may be located in a side surface of the charging device 3.

In this example, the charging device comprises a printed circuit board 39, on which a charging coil configured for generating a magnetic charging field for transferring power to a receiving coil of an implantable device (not shown) is located. Alternatively, the charging coil may encircle the opening 303 in the bottom casing element 302. Said charging coil is powered by an electrical power source 4. The charging device 3 further comprises a connection 30 from the battery to the circuit of the device. It also comprises a connection for connecting the external device and the charging device. In some embodiments of the present invention, to enable said transfer of electrical power, at least the bottom casing element 302 is preferably transparent to magnetic fields. In this example, the electrical output element is adapted for electrically contacting the electrical input element, e.g., when the external control device is immobilized by the receiving element, but alternatively, the electrical output element and the electrical input element may be wireless, e.g. through coupled coils, so that charging may be based on inductive charging. These coils may be inductively coupled to each other when the external control device is immobilized by the receiving element. It is to be noted that the external device could be a portable device such as for example a mobile phone. The system may be adapted for charging the portable device, e.g. by induction. The electrical output element may be powered by the electrical power source 4. However, the invention is not limited thereto, and instead, the charging device 3 may comprise a further electrical power source, different from the electrical power source 4, for powering the electrical output element. In other words, the electrical output element and the charging coil may be powered by different electrical power sources. The electrical power source 4 may be any type of power source, but is preferably a rechargeable power source. For example, the electrical power source 4 may be charged by inputting power through the electrical output element, e.g., using a USB connection, as is known to the person skilled in the art. In such an example, the electrical output element may have a double function, that is, of enabling charging of the external control device, and of the at least one power source of the charging device.
In some examples, the charging device may comprise an elastic band 32 for easy fit into wearing means, e.g., a belt (not shown).

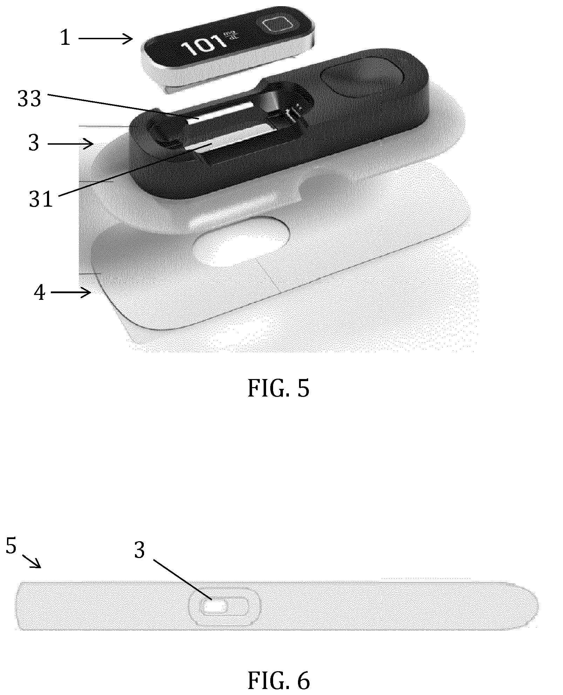

Reference is made to FIG. 5, which is a wearable system in according with embodiments of the present invention, comprising an external control device 1 and a charging device 3 that comprises an opening 31 comprising rails 33 on the walls for receiving the external control device 1. In this example, the wearable system further comprises a double sided adhesive 4, which may be made of silicon, for attaching the charging device 3 to a skin of a user, e.g., in the proximity of an implantable device (not shown). Thereby, the charging device 3 may simultaneously charge the implantable device and the external control device 1, while at the same time enabling communicative coupling between the external control device 1 and the implantable device. In this example, the charging device 3 comprises an elastic band 32 for easy fit into wearing means, e.g., a belt (not shown).

Reference is made to FIG. 6, which is a schematic view of a belt 5 coupled to a charging device 3. The belt may facilitate immobilisation of the charging device 3 on part of the skin of a user. Herein, the belt 5 comprises an opening enabling physically contacting the charging device 3 to the user wearing the belt 5 holding the charging device 3.

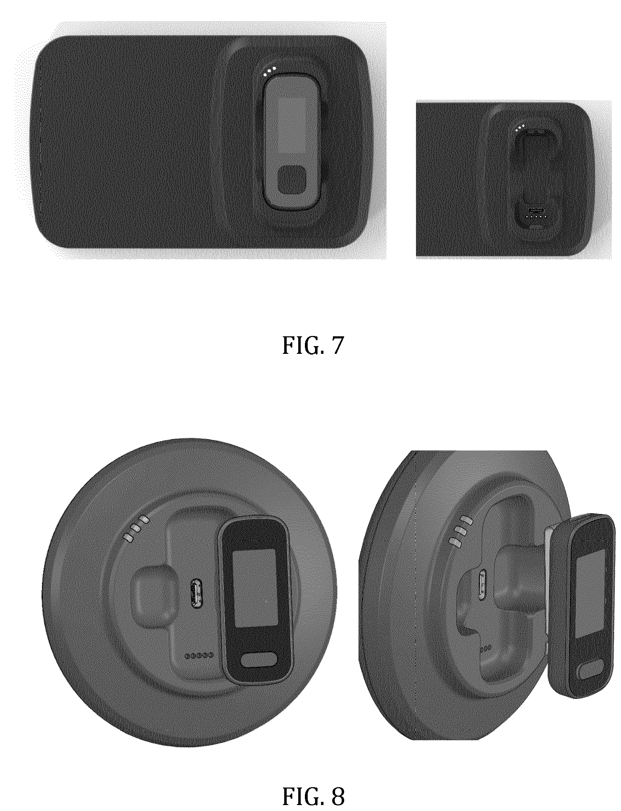

Further by way of illustration, FIG. 7 and 8 illustrate further examples of charging systems which can be applied with a belt or connected to clothes or to a body, allowing to charge an external device as well as an implanted sensor element. As illustrated, when positioned in the charging system, the external device may cover further connections for security, e.g. the connection for charging the charging system via an external source. In the example shown in FIG. 8, external charging of the charging system may be performed via a USB connection, although embodiments are not limited thereto and also other connection types may be used.

It is to be understood that although preferred embodiments, specific constructions and configurations, as well as materials, have been discussed herein for devices according to the present invention, various changes or modifications in form and detail may be made without departing from the scope of this invention. Steps may be added or deleted to methods described within the scope of the present invention.

## Claims

1. A wearable system comprising a charging device (3) for simultaneously charging an implantable device and an external control device (1) for reading out and/or controlling the implantable device,
the charging device (3) comprising at least one electrical power source (4), a first charging element for charging the implantable device and a second charging element for charging the external control device, the charging device (3) configured for simultaneously charging the implantable device and the external control device (1), wherein the at least one electrical power source itself is chargeable and wherein the system is configured to only allow mutually exclusive the charging of the at least one electrical power source on the one hand, and the charging of the implantable sensor and the external control device through the at least one electrical power source on the other hand.

2. The wearable system according to claim 1, wherein the system is configured such that positioning the external control device with respect to the second charging element prevents accessing a charging connection for charging the at least one electrical power source.

3. The wearable system according to any of the previous claims, wherein the first charging element is a charging coil configured for generating a charging field for transferring power from the electrical power source (4) to a receiving coil of the implantable device.

4. The wearable system according to any of the previous claims, wherein the second charging element is an electrical output element suitable for transferring electrical power from the electrical power source (4) to an electrical input element of the external control device (1).

5. The wearable system according to any of the previous claims, wherein the charging device (3) further comprises a receiving element configured for reversibly immobilizing the external control device (1) with respect to the charging device (3).

6. The wearable system according to any of the previous claims, wherein the charging device is configured for detecting whether the first charging element (3) is at a location with respect to the implantable device suitable for transferring power.

7. The wearable system according to any of the previous claims, further comprising a belt (5) wearable by a user, wherein the belt (5) is configured for holding the charging device (3), wherein the wearable system is configured for physically contacting the charging device (3) to the user wearing the belt (5).

8. The wearable system according to any of the previous claims, the wearable system further comprising the external control device (1),
wherein the external control device (1) comprises an electrical input element (21) that can be coupled to the second charging element for transferring power from the second charging element to the electrical input element (21), for charging the external control device (1).

9. The wearable system according to claim 8, wherein the external control device (1) comprises a fastening element configured for being received by a receiving element of the wearable system for immobilizing the external control device (1) with respect to the wearable system.

10. The wearable system according to any of claims 8 to 9, wherein the external control device (1) comprises an alarm element, and a control unit configured for determining, from data received by the external control device (1), whether to generate an alarm with the alarm element.

11. The wearable system according to any of the previous claims, the wearable system furthermore comprising the implantable device, for example one of an implantable sensor for sensing at least one metabolite in a body of a living creature, an implantable sensor for sensing a parameter of a living creature, a neurostimulator or an active orthopedics.

12. The wearable system according to claim 11, wherein the implantable device comprises an implantable sensor for sensing at least one metabolite, such as for example one or more of glucose, ketones, lactate, urea and/or lipids.

13. A method for simultaneously charging an implantable device and an external control device (1) by at least one electrical power source of a charging device in a wearable system, the method comprising:
- positioning the wearable system on the body so that the implantable device is positioned relative to a first charging element of the wearable system for allowing charging of the implantable device,
- coupling the external control device (1) to a second charging element of the wearable system for allowing charging of the external control device, and
- simultaneously charging the implantable device and the external control device through the wearable system,
the method further comprising only allowing mutually exclusive charging of the at least one electrical power source on the one hand, and charging of the implantable sensor and the external control device through the at least one electrical power source on the other hand.

14. The method according to claim 13, wherein the method further comprises charging at least one electrical power source of the wearable system when the external control device is not coupled to the wearable system.

## Patentansprüche

1. Ein am Körper tragbares System, das eine Ladevorrichtung (3) zum gleichzeitigen Laden einer implantierbaren Vorrichtung und einer externen Steuervorrichtung (1) zum Auslesen und/oder Steuern der implantierbaren Vorrichtung umfasst,
- wobei die Ladevorrichtung (3) mindestens eine elektrische Leistungsquelle (4), ein erstes Ladeelement zum Laden der implantierbaren Vorrichtung und ein zweites Ladeelement zum Laden der externen Steuervorrichtung umfasst, wobei die Ladevorrichtung (3) zum gleichzeitigen Laden der implantierbaren Vorrichtung und der externen Steuervorrichtung (1) konfiguriert ist, wobei die mindestens eine elektrische Leistungsquelle selbst geladen werden kann und wobei das System so konfiguriert ist, dass es wechselweise ausschließlich nur das Laden der mindestens einen elektrischen Leistungsquelle einerseits, und das Laden des implantierbaren Sensors und der externen Steuervorrichtung durch die mindestens eine elektrische Leistungsquelle andererseits ermöglicht.

2. Das am Körper tragbare System nach Anspruch 1, wobei das System derart konfiguriert ist, dass das Positionieren der externen Steuervorrichtung in Bezug auf das zweite Ladeelement das Zugreifen auf einen Ladeanschluss zum Laden der mindestens einen elektrischen Leistungsquelle verhindert.

3. Das am Körper tragbare System nach einem der vorstehenden Ansprüche, wobei das erste Ladeelement eine Ladespule ist, die zum Erzeugen eines Ladefeldes zum Übertragen von Leistung von der elektrischen Leistungsquelle (4) an eine Empfangsspule der implantierbaren Vorrichtung konfiguriert ist.

4. Das am Körper tragbare System nach einem der vorstehenden Ansprüche, wobei das zweite Ladeelement ein elektrisches Ausgangselement ist, das zum Übertragen von elektrischer Leistung von der elektrischen Leistungsquelle (4) an ein elektrisches Eingangselement der externen Steuervorrichtung (1) geeignet ist.

5. Das am Körper tragbare System nach einem der vorstehenden Ansprüche, wobei die Ladevorrichtung (3) weiter ein Empfangselement umfasst, das zum reversiblen Immobilisieren der externen Steuervorrichtung (1) in Bezug auf die Ladevorrichtung (3) konfiguriert ist.

6. Das am Körper tragbare System nach einem der vorstehenden Ansprüche, wobei die Ladevorrichtung konfiguriert ist zum Erkennen, ob sich das erste Ladeelement (3) an einer Stelle in Bezug auf die implantierbare Vorrichtung befindet, die für das Übertragen von Leistung geeignet ist.

7. Das am Körper tragbare System nach einem der vorstehenden Ansprüche, das weiter einen Gurt (5) umfasst, der von einem Benutzer am Körper getragen werden kann, wobei der Gurt (5) zum Halten der Ladevorrichtung (3) konfiguriert ist, wobei das am Körper tragbare System zum physischen Inkontaktbringen der Ladevorrichtung (3) mit dem Benutzer, der den Gurt (5) trägt, konfiguriert ist.

8. Das am Körper tragbare System nach einem der vorstehenden Ansprüche, wobei das am Körper tragbare System weiter die externe Steuervorrichtung (1) umfasst,
- wobei die externe Steuervorrichtung (1) ein elektrisches Eingangselement (21) umfasst, das zum Übertragen von Leistung vom zweiten Ladeelement zum elektrischen Eingangselement (21) zum Laden der externen Steuervorrichtung (1) mit dem zweiten Ladeelement gekoppelt werden kann.

9. Das am Körper tragbare System nach Anspruch 8, wobei die externe Steuervorrichtung (1) ein Befestigungselement umfasst, das zur Aufnahme durch ein Aufnahmeelement des am Körper tragbaren Systems zum Immobilisieren der externen Steuervorrichtung (1) in Bezug auf das am Körper tragbare System konfiguriert ist.

10. Das am Körper tragbare System nach einem der Ansprüche 8 bis 9, wobei die externe Steuervorrichtung (1) ein Alarmelement und eine Steuereinheit umfasst, die konfiguriert ist zum Bestimmen, anhand von Daten, die von der externen Steuervorrichtung (1) empfangen werden, ob mit dem Alarmelement ein Alarm erzeugt werden soll.

11. Das am Körper tragbare System nach einem der vorstehenden Ansprüche, wobei das am Körper tragbare System weiter die implantierbare Vorrichtung umfasst, zum Beispiel eines von einem implantierbaren Sensor zum Erfassen mindestens eines Metaboliten in einem Körper eines Lebewesens, einem implantierbaren Sensor zum Erfassen eines Parameters eines Lebewesens, einem Neurostimulator oder eines aktiven orthopädischen Geräts.

12. Das am Körper tragbare System nach Anspruch 11, wobei die implantierbare Vorrichtung einen implantierbaren Sensor zum Erfassen mindestens eines Metaboliten umfasst, wie zum Beispiel eines oder mehrere von Glukose, Ketonen, Laktat, Harnstoff und/oder Lipiden.

13. Ein Verfahren zum gleichzeitigen Laden einer implantierbaren Vorrichtung und einer externen Steuervorrichtung (1) durch mindestens eine elektrische Leistungsquelle einer Ladevorrichtung in einem am Körper tragbaren System, wobei das Verfahren umfasst:
- Positionieren des am Körper tragbaren Systems am Körper, sodass die implantierbare Vorrichtung relativ zu einem ersten Ladeelement des am Körper tragbaren Systems positioniert wird, um das Laden der implantierbaren Vorrichtung zu ermöglichen,
- Koppeln der externen Steuervorrichtung (1) mit einem zweiten Ladeelement des am Körper tragbaren Systems, um das Laden der externen Steuervorrichtung zu ermöglichen, und
- gleichzeitiges Laden der implantierbaren Vorrichtung und der externen Steuervorrichtung durch das am Körper tragbare System,
- wobei das Verfahren weiter das wechselseitige ausschließliche Laden nur der mindestens einen elektrischen Leistungsquelle einerseits und das Laden des implantierbaren Sensors und der externen Steuervorrichtung durch die mindestens eine elektrische Leistungsquelle andererseits ermöglicht.

14. Das Verfahren nach Anspruch 13, wobei das Verfahren weiter das Laden mindestens einer elektrischen Leistungsquelle des am Körper tragbaren Systems umfasst, wenn die externe Steuervorrichtung nicht mit dem am Körper tragbaren System gekoppelt ist.

## Revendications

1. Un système portable comprenant un dispositif de charge (3) pour charger simultanément un dispositif implantable et un dispositif de contrôle externe (1) pour lire et/ou contrôler le dispositif implantable, le dispositif de charge (3) comprenant au moins une source d'énergie électrique (4), un premier élément de charge pour charger le dispositif implantable et un second élément de charge pour charger le dispositif de contrôle externe, le dispositif de charge (3) étant configuré pour charger simultanément le dispositif implantable et le dispositif de contrôle externe (1), dans lequel ladite au moins une source d'énergie électrique elle-même est rechargeable et dans lequel le système est configuré pour ne permettre que de manière mutuellement exclusive la charge de ladite au moins une source d'énergie électrique d'une part, et la charge du capteur implantable et du dispositif de contrôle externe par ladite au moins une source d'énergie électrique d'autre part.

2. Le système portable selon la revendication 1, dans lequel le système est configuré de sorte que le positionnement du dispositif de contrôle externe par rapport au second élément de charge empêche l'accès à une connexion de charge pour charger ladite au moins une source d'énergie électrique.

3. Le système portable selon l'une quelconque des revendications précédentes, dans lequel le premier élément de charge est une bobine de charge configurée pour générer un champ de charge pour transférer de l'énergie de la source d'énergie électrique (4) à une bobine réceptrice du dispositif implantable.

4. Le système portable selon l'une quelconque des revendications précédentes, dans lequel le second élément de charge est un élément de sortie électrique adapté pour transférer de l'énergie électrique de la source d'énergie électrique (4) à un élément d'entrée électrique du dispositif de contrôle externe (1).

5. Le système portable selon l'une quelconque des revendications précédentes, dans lequel le dispositif de charge (3) comprend en outre un élément de réception configuré pour immobiliser de manière réversible le dispositif de contrôle externe (1) par rapport au dispositif de charge (3).

6. Le système portable selon l'une quelconque des revendications précédentes, dans lequel le dispositif de charge est configuré pour détecter si le premier élément de charge (3) est à un emplacement par rapport au dispositif implantable adapté pour transférer de l'énergie.

7. Le système portable selon l'une quelconque des revendications précédentes, comprenant en outre une ceinture (5) portable par un utilisateur, dans laquelle la ceinture (5) est configurée pour maintenir le dispositif de charge (3), dans lequel le système portable est configuré pour mettre en contact physique le dispositif de charge (3) avec l'utilisateur portant la ceinture (5).

8. Le système portable selon l'une quelconque des revendications précédentes, le système portable comprenant en outre le dispositif de contrôle externe (1), dans lequel le dispositif de contrôle externe (1) comprend un élément d'entrée électrique (21) qui peut être couplé au second élément de charge pour transférer de l'énergie du second élément de charge à l'élément d'entrée électrique (21), pour charger le dispositif de contrôle externe (1).

9. Le système portable selon la revendication 8, dans lequel le dispositif de contrôle externe (1) comprend un élément de fixation configuré pour être reçu par un élément de réception du système portable pour immobiliser le dispositif de contrôle externe (1) par rapport au système portable.

10. Le système portable selon l'une quelconque des revendications 8 à 9, dans lequel le dispositif de contrôle externe (1) comprend un élément d'alarme, et une unité de contrôle configurée pour déterminer, à partir des données reçues par le dispositif de contrôle externe (1), s'il faut générer une alarme avec l'élément d'alarme.

11. Le système portable selon l'une quelconque des revendications précédentes, le système portable comprenant en outre le dispositif implantable, par exemple un capteur implantable pour détecter au moins un métabolite dans le corps d'une créature vivante, un capteur implantable pour détecter un paramètre d'une créature vivante, un neurostimulateur ou une orthopédie active.

12. Le système portable selon la revendication 11, dans lequel le dispositif implantable comprend un capteur implantable pour détecter au moins un métabolite, tel que par exemple un ou plusieurs de glucose, cétones, lactate, urée et/ou lipides.

13. Un procédé pour charger simultanément un dispositif implantable et un dispositif de contrôle externe (1) par au moins une source d'énergie électrique d'un dispositif de charge dans un système portable, le procédé comprenant :
- positionner le système portable sur le corps de sorte que le dispositif implantable soit positionné par rapport à un premier élément de charge du système portable pour permettre la charge du dispositif implantable,
- coupler le dispositif de contrôle externe (1) à un second élément de charge du système portable pour permettre la charge du dispositif de contrôle externe, et
- charger simultanément le dispositif implantable et le dispositif de contrôle externe par le système portable,
le procédé comprenant en outre de ne permettre que de manière mutuellement exclusive la charge de ladite au moins une source d'énergie électrique d'une part, et la charge du capteur implantable et du dispositif de contrôle externe par ladite au moins une source d'énergie électrique d'autre part.

14. Le procédé selon la revendication 13, dans lequel le procédé comprend en outre la charge d'au moins une source d'énergie électrique du système portable lorsque le dispositif de contrôle externe n'est pas couplé au système portable.
